# EUROPEAN PATENT APPLICATION

(11) **EP 1 700 838 A1**
(43) Date of publication of application: **13.09.2006**
(21) Application number: 05425138.4
(22) Date of filing: 11.03.2005
(51) Int. Cl.: C07C 41/18, C07C 43/215

(54) **A process for the preparation of coenzyme Q10**

(71) Applicant: Gnosis S.p.A., 20033 Desio (MI) (IT)
(72) Inventor: Santambrogio, Gianni, 20092 Cinisello Balsamo (MI) (IT); Latanza, Augusto, 20020 Solaro (MI) (IT); Valoti, Ermanno, 24044 Dalmine (BG) (IT); Bianchi, Davide, 20100 Milano (IT)
(74) Representative: Minoja, Fabrizio

(57) **Abstract**

A process for the preparation of the compound of formula II 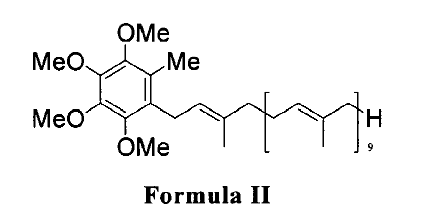 by removal of the phenylsulfone group from the compound of formula I 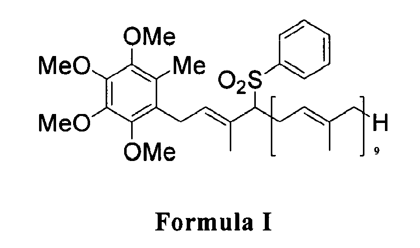 characterized in that the phenylsulfone group is removed in the presence of PdCl₂-1,2-diphenylphosphinoethane and LiHBEt₃.

## Description

The present invention relates to a process for the preparation of coenzyme Q10.

The invention further relates to a process for the preparation of the precursor of coenzyme Q10 by removing the phenylsulfone group from the intermediate of formula I

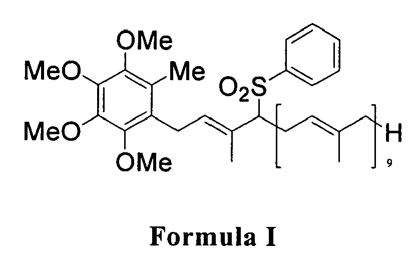

### Technological background

Coenzyme Q10 is a ubiquinone present in various animal and vegetable organisms, structurally related to vitamins K and E. Its main biological function concerns the transport of energy into the mitochondria. Coenzyme Q 10 also has antioxidizing and membrane stabilizing properties which make it useful as a dietetic supplement as well as pharmacologically active ingredient, in particular for the treatment of ischemic cardiovascular diseases.

Further therapeutical actions for coenzyme Q10 have been evidenced, which are useful in case of neurodegenerative diseases, ageing-related pathologies, decrease in immune defenses and the like.

A number of processes for the preparation of coenzyme Q 10 are known, involving different strategic approaches. One of said processes is disclosed in US 6,686,485 B2.

Fujita et al., 469-471 Synthesis (1981) disclose the preparation of coenzyme Q 10 starting from tetramethoxybromo toluene through formation of the corresponding Grignard, exchange with CuCl and subsequent coupling with (E)-4-chloro-2-methyl-1-phenylsulfonyl butene to give derivative A, which only contains one isoprene moiety. Condensation with solanesil bromide affords **compound** I

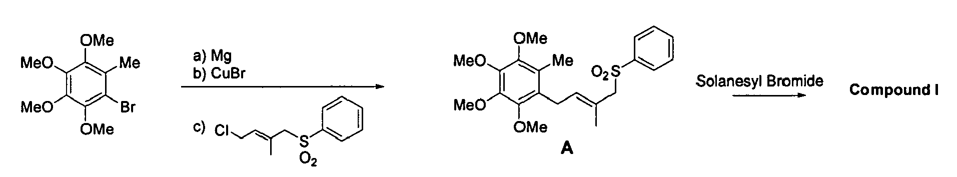

The subsequent desulfonation yields **compound** II. This process has, however, a drawback in that the Δ₃₋₄ isomer **(compound III)** can be formed in various percentages, which can be measured by NMR analysis and can range from 2 to 40% depending on the methods used. Among these methods, the use of sodium in ethanol; Li in ethylamine; sodium borohydride in the presence of Pd(PPh₃)4 or PdCl₂(PPh₃)₂; LiHBEt₃ in the presence of the above cited Pd catalysts and others such as PdCl₂(dppp) (dppp = 1,2-diphenylphosphinopropane), can be mentioned.

According to Mitsunobu M. et al., Chemistry Letters 1177-80 (1986), the phenylsulfonic group is regioselectively and quantitatively removed with the use of PdCl₂(dppp). There is however evidence that PdCl₂(dppp) cannot provide regiospecific reduction. In fact, as described, the reduction proceeds quantitatively but is accompanied by formation of approx. 15% of the Δ₃₋₄ isomer.

Experiments carried out by the Applicant, in which Pd had been replaced by Ni, namely using NiCl₂(dppp) with LiHBEt₃, were unsuccessful.

Mohri et al. Chemistry Letters 451-4 (1985) and Chemistry Letters 1177-80 (1986) disclose the desulfonylation of allyl phenylsulfones using different platinum phosphine complexes in the presence of hydrides. The reported data confirm that the most efficient catalyst is PdCl₂(dppp) whereas PdCl₂(dppe) (dppe=diphenylphosphinoethane) proves markedly less effective and substantially useless for practical purposes, giving yields of approx. 33%.

### Disclosure of the invention

Contrary to what reported in literature, it has now surprisingly been found that the catalyst PdCl₂(dppe) together with LiHBEt₃ (superhydride) is an excellent system for the removal of the phenylsulfone group both in terms of regioselective reduction (98% of desired isomer) and chemical yield (above 95%).

The work-up of the reaction, followed by crystallization of the formed compound, provides **compound** II in high yields (about 90%) as well as high isomeric purity (>99%).

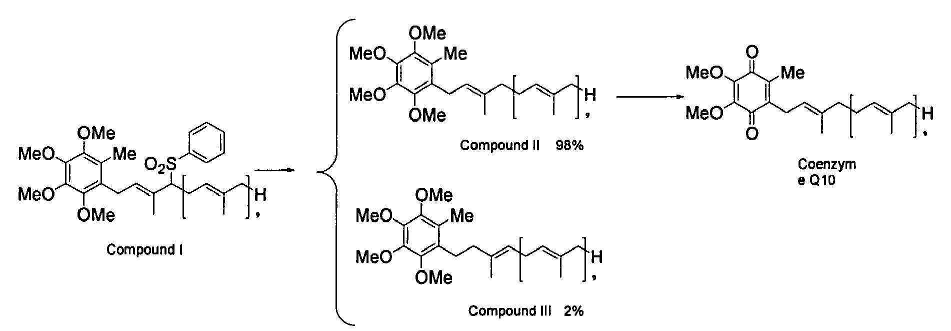

The reaction is carried out in an inert solvent such as tetrahydrofuran (THF), hexane, cyclohexane, ethyl ether and isopropyl ether, preferably tetrahydrofuran, at a temperature ranging from -20°C to room temperature.

After completion of the reaction, water is added, the lithium salts are filtered off, the solution is evaporated to dryness and the residue is taken up in a hydrocarbon such as cyclohexane, pentane, hexane, heptane, isomers thereof and/or mixtures thereof.

Pd can then be quantitatively recovered by addition of charcoal, filtration and subsequent combustion.

The resulting crude product, which contains at most 2.5-3% of the Δ₃₋₄ isomer, can be crystallized from methanol, ethanol, isopropanol or acetonitrile.

The PdCl₂(dppe) catalyst is used in molar ratios ranging from 1 to 10%, preferably 5%, while the Superhydride is used in molar ratios of 2 to 3, preferably 2.2 moles, per mole of **compound** I.

Lithium can be quantitatively recovered in the form of the phenylsulfinate which precipitates in the reaction medium.

A further object of the invention relates to the process for the preparation of coenzyme Q 10 by oxidation of the compound of formula II with cerium ammonium nitrate in acetonitrile solution in the presence of stoichiometric amounts of water.

Oxidation of compound II with cerium ammonium nitrate (CAN) was already described by Peyton J. et al., J. Org. Chem. 41 3627 (1976) using water as the solvent.

The process of the invention, using acetonitrile or THF as the solvent and water in preferably stoichiometric amounts (2 moles) or in an excess not higher than 4 moles per mole of compound II, provides the reaction product with higher purity and yield. A remarkable advantage of this procedure on an industrial scale, is that all of the cerium salts are insoluble and therefore can be quickly recovered by simple filtration from the solvent mixture used, upon completion of the reaction.

The moles of CAN used range from 2 to 4, preferably 3, per mole of the product.

The invention is described in greater detail in the following examples.

### Example I (deprotection)

THF (1500 ml), compound I (147 g) and the catalyst (PdCl₂(dppe) (4 g) are placed in a 4-necked round-bottom 2000 ml flask, equipped with thermometer, under nitrogen stream. The mixture is stirred for 10' then cooled to -5°C. The LiHBEt₃ solution (20% superhydride solution in THF, 157 ml) is dropped therein, in about 30' at 0°C. The mixture is stirred for a further 6 hours at 0-5°C, then 6.8 ml of water are dropped therein and after 15' the formed salts (17.36 g of lithium phenylsulfinate) are filtered off. The mixture is concentrated to dryness, then added with 1000 ml of cyclohexane, 40 g of charcoal and left under stirring for 30'. The mass is filtered (the charcoal contains the catalyst which can be recovered) to obtain an almost colourless clear solution. This is concentrated to obtain 125 g of a pale yellow oil which is crystallized from methanol (850 ml). Upon filtration, 119 g of solid white product (m.p. 37-38°C) are obtained, consisting of compound I (having isomeric purity higher than 99%).

### Example II (oxidation)

CAN (33.15 g) suspended in acetonitrile (150 ml) and water (725 mg, 2 moles per mole of product, depending on the reaction stoichiometry) are placed into a round-bottom 3-necked 500 ml flask. After complete dissolution of CAN, the solution is cooled to -5°C and **compound** I (18 g) dissolved in dichloromethane (79 ml) is dropped therein, in 15'. Temperature reaches -2°C. The mixture is left under stirring at 0-3°C for 60', then added with dichloromethane (180 ml) and stirred for 2'. The precipitated salts are filtered off and washed with approx. 70 ml of dichloromethane. The filtered salts contain all the starting cerium used, but in trivalent form (thus allowing the quick recovery of the salts as well as less costs of smaltimento of the aqueous solutions used for the washings upon completion of the reaction). The combined organic phases are washed with water (180 ml), then with a 2.5% sodium bicarbonate solution (90 ml) and finally with NaCl 20% aqueous solution (72 ml). Concentration under vacuum at 30°C affords a yellow-orange oil (17.6 g) having Q10 content of approx. 78%. The product, coenzyme Q10, can be recovered by purification chromatography (13.2 g) or by crystallization from ethanol (12 g).

## Claims

1. A process for the preparation of the compound of formula II 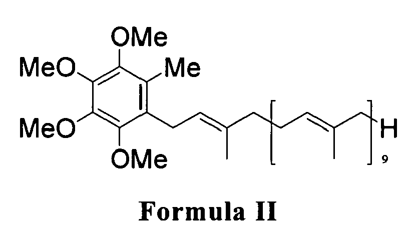 by removal of the phenylsulfone group from the compound of formula I 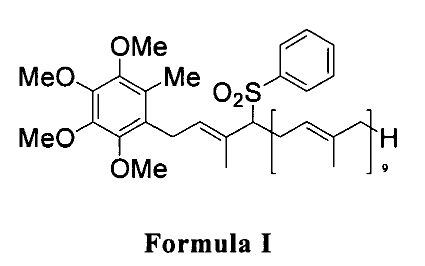 **characterized in that** the phenylsulfone group is removed in the presence of PdCl₂-1,2-diphenylphosphinoethane and LiHBEt₃.

2. A process as claimed in claim 1 wherein the reaction is carried out in tetrahydrofuran, hexane, cyclohexane, ethyl ether and isopropyl ether.

3. A process as claimed in claim 1 or 2 in which the product is crystallized from methanol, ethanol, isopropanol or acetonitrile.

4. A process as claimed in any one of claims 1 to 3 in which PdCl₂(dppe) is used in molar ratios ranging from 1 to 10%, preferably 5%, while Superhydride is used in molar ratios from 2 to 3, preferably 2.2 moles, per mole of compound I.

5. A process for the preparation of coenzyme Q10 by oxidation of the compound of formula II with cerium ammonium nitrate in acetonitrile or THF solution in the presence of water in amounts ranging from 2 to 4 moles per mole of compound II.

6. A process as claimed in claim 5 in which the compound of formula II is obtained according to the process of claim 1.

7. A process as claimed in claim 5 or 6 in which the product is crystallized from ethanol, methanol or isopropanol.

8. A process as claimed in any one of claims 5 to 7 in which cerium ammonium nitrate is used in amounts ranging from 2 to 4 moles per mole of compound II, preferably 3 moles per mole of compound II.
